# EUROPEAN PATENT APPLICATION

(11) **EP 4 714 459 A1**
(43) Date of publication of application: **25.03.2026**
(21) Application number: 24807632.5
(22) Date of filing: 17.05.2024
(51) Int. Cl.: A61K 35/24, A61P 43/00, A61P 29/00, C12N 5/071

(54) **COMPOSITION FOR PREVENTING OR TREATING SALIVARY GLAND DISEASES, COMPRISING EPITHELIAL PROGENITOR CELLS OR STEM CELLS IN MINOR SALIVARY GLAND DUCT**

(30) Priority: 17.05.2023 KR 20230064037
(71) Applicant: UIF (University Industry Foundation), Yonsei University, Seoul 03722 (KR)
(72) Inventor: LIM, Jae-Yol, Seoul 06229 (KR); YOON, Yeo-Jun, Seoul 07339 (KR); JEONG, Yejin, Seoul 04706 (KR); HONG, Yongpyo, Seoul 03988 (KR)
(74) Representative: Calysta NV
(86) International application number: PCT/KR2024/095778
(87) International publication number: WO 2024/237762

(57) **Abstract**

The present invention is a technology related to a pharmaceutical composition for preventing or treating salivary gland diseases, comprising epithelial progenitor cells or stem cells derived from minor salivary gland ducts. The present invention provides, for a therapeutic purpose, epithelial progenitor cells or stem cells derived from monoclonal minor salivary gland ducts so as to stably mitigate inflammation in salivary glands and improve a saliva production function in the human body, and thus can effectively treat salivary gland diseases.

## Description

### [Technical Field]

The present invention relates to a pharmaceutical composition for preventing or treating salivary gland diseases, comprising epithelial progenitor cells or stem cells derived from minor salivary gland ducts.

### [Background Art]

Salivary gland diseases are conditions in which the normal function of the salivary glands is impaired as a result of damage or inflammation in the salivary gland tissue. Examples of the salivary gland diseases include xerostomia, infectious acute sialadenitis, infectious chronic sialadenitis, salivary gland tuberculosis, Sjogren's syndrome, sialolithiasis, salivary duct stenosis, sialadenosis, salivary gland tumors, salivary hypofunction due to aging, etc.

Among salivary gland diseases, Sjogren's syndrome is a type of autoimmune disease that mainly occurs in middle-aged and older women. It is a disease in which lymphocytes invade glandular organs in the body, such as the salivary glands, lacrimal glands, and pancreas, for unknown reasons. A representative symptom is irreversible dry mouth caused by decreased salivary gland function. Clinical trials utilizing monoclonal antibodies, which are commonly employed in the treatment of various autoimmune diseases, are underway to treat Sjogren's syndrome, but no effective results have been reported to date.

In other words, currently, there is no curative treatment for Sjogren's syndrome or any method for maintaining long-term improvement, leaving symptomatic treatment with artificial saliva and pylocarpine as the only available options.

Regarding this issue, numerous research and development efforts are underway on treatment methods using cell therapy, etc. However, cell therapy products still face challenges, such as intercellular heterogeneity and cell aging, related to their quality (proliferation rate, telomere length, pluripotency markers, and multilineage differentiation capacity). Accordingly, clinical trials based on these cells have progressed considerably slowly.

Therefore, the present inventors have demonstrated that selectively cultured and proliferated novel epithelial progenitor cells or stem cells derived from minor salivary gland ducts can be used to alleviate salivary gland inflammation and normalize saliva production function, thereby completing the present invention.

### [Disclosure]

### [Technical Problem]

An object of the present invention is to provide a pharmaceutical composition for preventing or treating salivary gland diseases, comprising epithelial progenitor cells or stem cells derived from minor salivary gland ducts.

### [Technical Solution]

To avoid confusion arising from overlapping content, redundant descriptions will be omitted below. In other words, the invention is not limited to the foregoing description and should be construed in accordance with the overall content of the invention.

The present invention provides a pharmaceutical composition for preventing or treating salivary gland diseases, comprising epithelial progenitor cells or stem cells derived from minor salivary gland ducts.

As used herein, the term "salivary gland" refers to an organ that produces and secretes saliva.

The salivary glands are classified into major salivary glands, such as the parotid gland, submandibular gland (submaxillary gland), and sublingual gland, and minor salivary glands distributed in various parts of the oral mucosa, such as mucous glands that exist in the mucous membrane of the oral cavity.

In the present invention, the term "salivary gland duct" refers to a conduit through which saliva is transported from the salivary glands to the oral cavity, and includes the parotid duct (Stensen duct), the submandibular duct (Wharton duct), the sublingual duct (Rivinus duct), etc.

As used herein, "progenitor cell" refers to a cell committed to differentiation into a particular cell type or to the formation of a specific tissue.

As used herein, "stem cell" refers to an undifferentiated cell capable of differentiating into various types of tissue. Stem cells may self-renew through repeated division and may also differentiate into cells with specific functions depending on the environment.

As used herein, "epithelial" refers to a condition or characteristic of being composed of, or pertaining to, epithelium, a membrane tissue that covers internal and external body surfaces.

The epithelial progenitor cells or stem cells according to the present invention may be derived from the major salivary gland or the minor salivary gland, preferably from the minor salivary gland. In the present invention, the term "epithelial progenitor cells or stem cells" in the minor salivary gland ducts is interpreted to encompass not only the epithelial progenitor cells or stem cells derived from the minor salivary gland ducts, but also all of them.

Specifically, the term "epithelial progenitor cells or stem cells" in the minor salivary gland ducts according to the present invention refers to progenitor cells derived from ducts that are distributed in various places in the mouth and secrete saliva in small amounts. Specifically, the cells may be capable of self-renewal and differentiation into cells present in the ducts of the minor salivary gland epithelial parenchyma. The epithelial progenitor cells or stem cells derived from the minor salivary gland ducts may be cells capable of differentiating into other salivary gland cells derived from the salivary gland epithelium parenchyma.

The term "differentiation" may refer to a phenomenon in which a cell's structure or function becomes specialized during cell division, proliferation, and growth. More specifically, the epithelial progenitor cells or stem cells derived from the minor salivary gland ducts may differentiate into basal cells, luminal ductal cells, glandular cells, and myoepithelial cells derived from the salivary gland parenchyma.

More specifically, the epithelial progenitor cells or stem cells derived from the minor salivary gland ducts according to the present invention may comprise salivary gland basal cells of CD49f⁺ and CD26⁻.

Therefore, the epithelial progenitor cells or stem cells derived from the minor salivary gland ducts according to the present invention include cells with characteristics of salivary gland epithelial tissue and also cells with characteristics of salivary gland basal cells. Further, the cells according to the present invention exhibit excellent characteristics in that they possess multipotent differentiation potential into basal, luminal, acinar, and myoepithelial cells, which are parenchymal cells derived from the salivary gland, thereby exhibiting recovery potential for the salivary gland.

The epithelial progenitor cells or stem cells derived from the minor salivary gland ducts according to the present invention may be cultured by treating the epithelial progenitor cells or stem cells derived from the minor salivary gland ducts in a medium composition containing a Rho-associated protein kinase (ROCK) inhibitor, a transforming growth factor-beta (TGF-β) inhibitor, and a bone morphogenetic protein (BMP) inhibitor.

The epithelial progenitor cells or stem cells derived from the minor salivary gland ducts may be cells derived from minor salivary gland tissue.

In the present invention, a chemically induced reprogramming method may be applied to selectively isolate homogeneous cells, i.e., epithelial progenitor cells or stem cells derived from the minor salivary gland ducts, and simultaneously a medium composition suitable for their survival and proliferation may be applied to isolate and utilize only cell sources suitable for treatment. Moreover, it is possible to obtain a large amount of desired cells in a short period of time without the step of manufacturing a Working Cell Bank (WCB) through rapid proliferation of monoclonal cells of epithelial progenitor cells or stem cells derived from the minor salivary gland ducts, thereby providing cells suitable for rapid clinical application and a manufacturing method thereof.

The minor salivary gland tissue according to the present invention may be derived from any individual, preferably a human. Preferably, it may be derived from an adult human. More preferably, it may be autologous minor salivary gland tissue. The minor salivary gland tissue may be isolated from a small amount of tissue, 1-2 mm² in size, obtained during a tissue biopsy. Under the chemically defined culture conditions of the present invention, it is possible to isolate and culture sufficient amounts of salivary gland epithelial ductal progenitor cells or stem cells. Specifically, target cells may be sufficiently obtained in a desired amount through culturing using chemically induced reprogramming using a combination of small molecule compounds.

The culturing any cells induced from the minor salivary gland may be performed in the presence of the ROCK inhibitor, the TGF-beta inhibitor, and the BMP inhibitor.

The ROCK inhibitor acts to inhibit the activity of serine/threonine kinase that functions as a target protein for Rho (Rho A, Rho B and Rho C), and may be R-(+)-trans-4-(1-Aminoethyl)-N-(4-pyridyl)cyclohexane carboxamide dihydrochloride monohydrate (Y-27632), Fasudil or H-1152. Preferably, the ROCK inhibitor may be Y-27632. More preferably, the ROCK inhibitor may be 1 to 20 µM of Y-27632.

The TGF-beta inhibitor is any substance that inhibits the function of the TGF-beta receptor, such as a protein, peptide, or small molecule, and may be A83-01, SB-431542, SB-505124, SB-525334, SD-208, RepSOX, LY-36494, and/or SJN-2511. Preferably, the TGF-beta inhibitor may be A83-01. More preferably, the TGF-beta inhibitor may be 0.2 to 2 µM of A83-01.

The BMP inhibitor is an agent that binds to a BMP molecule to form a complex, binds to a BMP receptor, and prevents the binding of a BMP ligand to the receptor, such as an antibody that binds to the receptor. The BMP inhibitor may be a protein or a small molecule, and may be natural, modified, and/or partially or fully synthetic. In addition, the BMP inhibitor may be Noggin, Dorsomorphin, Gremlin 1, DMH1, and/or LDN-193189. Preferably, the BMP inhibitor may be DMH1 or LDN-193189. More preferably, the medium composition may include DMH1 at a final concentration of 0.1 to 2 µM or LDN193189 at a final concentration of 0.05 to 0.5 µM.

Preferably, the culture may be performed under Y-27632, A83-01, and DMH1 or LDN193189.

Preferably, the culture may be performed under Y-27632, A83-01, and DMH1.

Specifically, the culture may be performed under Y-27632, A83-01, and DMH1. If necessary, antibiotics may be further contained in the culture medium.

The culture medium described above may be a conventional basal medium. The basal medium may be any basal medium suitable for animal or human cells, subject to the limitations provided herein.

The basal medium for animal or human cell culture typically contains a number of components necessary to support the maintenance of the cultured cells. Suitable combinations of components may be readily formulated by those skilled in the art, taking into account the following. The basal medium for use in the present invention may generally comprise nutrient solutions containing culture components known from the literature and standard cell culture components, such as amino acids, vitamins, lipid supplements, mineral salts, carbon energy sources, and buffers. In some embodiments, the culture medium is further supplemented with one or more standard cell culture components selected from, for example, amino acids, vitamins, liquid supplements, mineral salts, carbon energy sources, and buffers.

Those skilled in the art will understand, based on common knowledge, the types of culture media capable of being used as base media in the differentiation media of the present invention. Potentially suitable cell culture media are commercially available and include, but are not limited to, Dulbecco's Modified Eagle Medium (DMEM), Minimum Essential Medium (MEM), KnockOut^{™} DMEM (KO-DMEM), Glasgow's Minimum Essential Medium (G-MEM), Basal Medium Eagle (BME), DMEM/F-12, Advanced DMEM/F-12, Iscove's Modified Dulbecco's Medium (IMDM), Ham's F-10, Ham's F-12, Medium 199, and RPMI 1640.

Epithelial cell culture media may also be used. Specifically, epithelial cell culture media such as CNT20, Cnt50, CnT-PR, KSFM, and DermaCult may be used. For example, the basal medium may include DermaCult^{™} Keratinocyte Expansion Medium or CnT-PR (CELLnTEC) Medium. For example, the basal medium may be DermaCult^{™} Keratinocyte Expansion Medium or CnT-PR (CELLnTEC) Medium. More preferably, it may be CnT-PR (CELLnTEC) Medium.

If necessary, Prinmocin^{™} and/or Dermacult Supplement^{™} may be further included.

The epithelial progenitor cells or stem cells derived from the minor salivary gland ducts according to the present invention may be produced by: (1) culturing (preferably chemically induced reprogramming) minor salivary gland-derived cells in a medium composition containing a ROCK inhibitor, a TGF-beta inhibitor, and a BMP inhibitor; and (2) culturing and proliferating the epithelial progenitor cells or stem cells derived from the minor salivary gland ducts cultured and isolated in Step (1) in a medium containing the ROCK inhibitor, the TGF-beta inhibitor, and the BMP inhibitor.

More specifically, the cells according to the present invention may be produced by (1) culturing (preferably, chemically induced reprogramming) cells derived from the minor salivary gland in a medium composition containing Y-27632, A83-01, and DMH1 or LDN193189; and (2) culturing and proliferating the epithelial progenitor cells or stem cells derived from the minor salivary gland ducts cultured and isolated in Step (1) in a medium containing Y-27632, A83-01, and DMH1 or LDN193189.

More specifically, the cells according to the present invention may be produced by (1) culturing (preferably, chemically induced reprogramming) cells derived from the minor salivary gland in a medium composition containing Y-27632, A83-01, and DMH1; and (2) culturing and proliferating the epithelial progenitor cells or stem cells derived from the minor salivary gland ducts cultured and isolated in Step (1) in a medium containing Y-27632, A83-01, and DMH1.

More specifically, the cells according to the present invention may be produced by (1) culturing (preferably, chemically induced reprogramming) cells derived from the minor salivary gland in a medium composition containing Y-27632, A83-01, and LDN193189; and (2) culturing and proliferating the epithelial progenitor cells or stem cells derived from the minor salivary gland ducts cultured and isolated in Step (1) in a medium containing Y-27632, A83-01, and LDN193189. The cells derived from the minor salivary glands according to the present invention may be obtained from the tissue of the minor salivary gland. Since the minor salivary gland tissue is in a tissue-formed state, various cells exist in the tissue. In other words, when passage 1 (P1) cells in a proliferated state are used, the cells do not exhibit uniform characteristics due to the mixing of different cell types, and these non-uniform cells are not desirable for clinical applications. Therefore, the present invention provides epithelial progenitor cells or stem cells derived from the minor salivary gland ducts having uniform characteristics by obtaining the epithelial progenitor cells or stem cells derived from the minor salivary gland ducts under the culture composition described above and selectively culturing and proliferating the obtained cells.

The epithelial progenitor cells or stem cells derived from the minor salivary gland ducts may stably form organoids through 3D culture. Unlike cells cultured using conventional subculture methods, the epithelial progenitor cells or stem cells exhibit extremely rapid population doubling even in 2D culture, allowing for the easy acquisition of large numbers of cells, and further, exhibit very low levels of cell aging, superior proliferation and differentiation potential, and excellent safety in the human body.

The epithelial progenitor cells or stem cells derived from the minor salivary gland ducts highly express any one or more cell surface markers selected from the group consisting of CD29, CD44, CD49f, CD146, and CD166. Furthermore, the cells according to the present invention do not express, or express at low levels, any one or more cell surface markers selected from the group consisting of CD24, CD26, CD34, CD90, and CD117.

In addition, the cells according to the present invention contain a high proportion of CD49f⁺ and CD26⁻ salivary gland basal cells.

According to an embodiment, the epithelial progenitor cells or stem cells derived from the minor salivary gland duct according to the present invention may be characterized by expressing KRT5 and/or E-Cadherin. KRT5 corresponds to a marker of salivary gland basal ductal cells, and E-Cadherin corresponds to a marker of salivary gland epithelial cells.

Furthermore, the epithelial progenitor cells or stem cells derived from minor salivary gland ducts according to the present invention may be characterized by expressing at least one selected from the group consisting of KRT5 and KRT19. KRT5 and KRT19 correspond to markers of salivary gland ductal basal cells or salivary gland ductal progenitor cells.

Further, the epithelial progenitor cells or stem cells derived from the minor salivary gland ducts according to the present invention may be characterized by not expressing at least one selected from the group consisting of CDKN2A, Fibronectin-1, and Vimentin. CDKN2A is a senescent cell marker, and Fibronectin-1 and Vimentin are mesenchymal stem cell markers.

More preferably, the culturing of the epithelial progenitor cells or stem cells derived from the minor salivary gland ducts according to the present invention may further include a step of proliferating monoclonal cells of the epithelial progenitor cells or stem cells derived from the minor salivary gland ducts. This is preferably performed in the presence of a Rho-associated protein kinase (ROCK) inhibitor, a transforming growth factor-beta (TGF-β) inhibitor, and a bone morphogenetic protein (BMP) inhibitor.

In clinical applications, it is crucial to obtain a sufficient number of cells exhibiting comparable therapeutic properties. Therefore, when using the culture method in the presence of the ROCK inhibitor, the TGF-beta inhibitor, and the BMP inhibitor according to the present invention, it is possible to provide a large amount of (monoclonal) cells capable of safely differentiating into minor salivary gland cells from a small amount of salivary gland tissue, within a short period of time, through a two-dimensional culture method, while minimizing DNA damage and suppressing aging, thereby effectively maintaining differentiation potential.

This cell proliferation step involves seeding and culturing monoclonal stem cells in a medium at a cell density of 50 to 1,000 cells/cm². This may induce rapid monoclonal stem cell proliferation, enabling rapid yield of the final product. Further, cells obtained in this manner exhibit excellent efficacy in the treatment of salivary gland diseases.

In particular, this method offers the advantage of providing cells with excellent proliferative potential and therapeutic efficacy even under xeno-free, serum-free, and feeder-free conditions.

To obtain the epithelial progenitor cells or stem cells derived from the minor salivary gland ducts from the minor salivary gland tissue, a subfractionation culturing method (SCM) may be preferably used, but is not limited thereto.

In the present invention, "subfractionation culture method" refers to a method of isolating cells based on specific gravity. This refers to a process in which cells are first extracted from the above-described minor salivary gland tissue or minor salivary gland ducts, cultured in a cell culture medium, and then only the supernatant is collected, transferred to a culture vessel with or without a coating agent, and cultured, and the same process is repeated several times. This subfractionation culture method is characterized by the repeated process of repeatedly obtaining and culturing the supernatant without a centrifugation process, and ultimately has the advantage of being able to obtain minor salivary gland ductal progenitor cells or stem cells without contamination of other cells. In particular, by obtaining monoclones of the epithelial progenitor cells or stem cells derived from minor salivary gland ducts having epithelial stem cell characteristics during this process, only cells with excellent therapeutic efficacy for salivary gland diseases may be provided.

In the present invention, the subfractionation culture method may specifically include a) transferring the supernatant from a first container to a second container; and b) culturing the cells present in the second container to obtain a supernatant. More specifically, the steps of: a) transferring the supernatant from a first container to a second container; and b) culturing the cells present in the second container to obtain a supernatant may be repeated at least once to five times, for example, once, twice, three times, four times, or five times.

The present invention provides epithelial progenitor cells or stem cells derived from the minor salivary gland ducts cultured in a medium composition containing a Rho-associated protein kinase (ROCK) inhibitor, a transforming growth factor-beta (TGF-β) inhibitor, and a bone morphogenetic protein (BMP) inhibitor.

More specifically, the present invention provides monoclonal cells of epithelial progenitor cells or stem cells derived from minor salivary gland ducts, produced by (1) subfractionation culturing of cells derived from the minor salivary gland in a medium composition containing a Rho-associated protein kinase (ROCK) inhibitor, a transforming growth factor-beta (TGF-β) inhibitor, and a bone morphogenetic protein (BMP) inhibitor; and (2) culturing and proliferating the isolated epithelial progenitor cells or stem cells derived from the minor salivary gland ducts in a medium composition containing a ROCK inhibitor, a TGF-beta inhibitor, and a bone morphogenetic protein (BMP) inhibitor.

The monoclonal cells of epithelial progenitor cells or stem cells derived from the minor salivary gland ducts have the characteristics of epithelial stem cells and multipotency to differentiate into various cells in the salivary gland. The cells also exhibit the expression characteristics of the above-described markers.

The present invention provides a pharmaceutical composition comprising the epithelial progenitor cells or stem cells derived from the minor salivary gland ducts.

The present invention provides a pharmaceutical composition for preventing or treating salivary gland diseases, comprising the epithelial progenitor cells or stem cells derived from the minor salivary gland ducts.

In particular, the composition according to the present invention may be utilized for therapeutic purposes as a bioengineering technique for restoring cell or tissue function. For example, the pharmaceutical composition may be utilized as a transplant material and applied to the treatment of various salivary gland diseases. In particular, the pharmaceutical composition may be considered as a material for regenerating and reconstructing damaged (including dysfunctional) salivary gland tissue. Therefore, the pharmaceutical composition according to the present invention may be a tissue therapeutic agent.

As used herein, "salivary gland disease" refers to a condition in which the normal function of the salivary glands is impaired due to damage to the salivary gland tissue or the occurrence of inflammation, and includes xerostomia, infectious acute sialadenitis, infectious chronic sialadenitis, salivary gland tuberculosis, Sjogren's syndrome, sialolithiasis, salivary duct stenosis, sialadenosis, salivary gland tumors, salivary hypofunction due to aging, salivary hypofunction due to medication, and salivary hypofunction due to radiation therapy. Preferably, the salivary gland disease may be Sjogren's syndrome. Sjogren's syndrome is a type of autoimmune disease that mainly occurs in middle-aged and older women, and refers to a disease in which lymphocytes infiltrate glandular organs in the body, such as the salivary glands, tear glands, and pancreas, due to unknown reasons.

The term "prevention" as used herein refers to any action that inhibits or delays the onset of a salivary gland disease by administering a pharmaceutical composition according to the present invention.

The term "treatment" as used herein refers to any action that improves or beneficially alters the symptoms of a salivary gland disease by administering the composition according to the present invention.

The composition according to the present invention exhibits excellent preventive and therapeutic efficacy against diseases by possessing direct differentiation, immunomodulatory, antifibrotic, and growth factor paracrine effects.

According to an embodiment, the composition according to the present invention may restore salivary gland function by dose-dependently restoring salivary secretion to normal levels and reducing damage to salivary gland ducts.

The pharmaceutical composition of the present invention may be formulated and provided in an appropriate form. In addition to the above-described effective ingredient, cells, the composition may be prepared by containing one or more pharmaceutically acceptable carriers.

Further, the pharmaceutical composition of the present invention may be formulated and administered as a unit dosage form of pharmaceutical preparation suitable for administration into the body of a patient according to conventional methods in the pharmaceutical field, wherein the preparation may contain an effective amount for single or multiple administrations. Suitable dosage forms for this purpose include parenteral preparations such as injections and infusions. The dosage of the pharmaceutical composition may vary depending on the patient's age, weight, sex, dosage form, health status, and disease severity. The composition may be administered once or several times daily at regular intervals, as determined by a physician or pharmacist.

The route and method of administration of the pharmaceutical composition of the present invention are independent and not particularly limited. Any route and method of administration may be employed, provided that the pharmaceutical composition can reach the target site.

Preferably, the pharmaceutical composition of the present invention may be administered directly into a duct, preferably a salivary gland duct, of a patient requiring treatment.

If necessary, the pharmaceutical composition is administered with an extracellular matrix. The extracellular matrix includes, but is not limited to, fibrin, laminin, collagen, and/or alginate. Examples of extracellular matrix-producing cells include chondrocytes, which primarily produce collagen and proteoglycans; fibroblasts, which primarily produce type IV collagen, laminin, interstitial procollagen, and fibronectin; and colonic myofibroblasts, which primarily produce collagen (types I, III, and V), chondroitin sulfate proteoglycans, hyaluronic acid, fibronectin, and tenascin-C. These are "naturally occurring ECMs." The naturally occurring ECMs may be commercially available. Examples of commercially available extracellular matrices include extracellular matrix proteins (Invitrogen) and basement membrane preparations from Engelbreath-Holm-Swarm (EHS) mouse sarcoma cells (such as Cultrex^{®} basement membrane extract (Trevigen, Inc.), type I collagen (Invitrogen), Vitrogel^{®} (TheWell Bioscience Inc.), or Matrigel^{®} (BD Biosciences)). In other words, the composition according to the present invention may be embedded in the above-described support and used for transplantation, or mixed and used for transplantation, as needed.

Further, dimethyl sulfoxide (DMSO), etc., may be added for cell protection, and antibiotics, etc., may be added for inhibiting bacterial contamination. Various components (vitamins, cytokines, growth factors, steroids, etc.) may also be added to the transplant material of the present invention for inducing cell activation, proliferation, or differentiation.

The pharmaceutical composition of the present invention may include conventional pharmaceutically acceptable inert carriers and diluents. Pharmaceutically acceptable carriers and diluents that may be included in the pharmaceutical composition of the present invention include, but are not limited to, excipients such as starch, sugar, and mannitol; fillers and extenders such as calcium phosphate; cellulose derivatives such as carboxymethylcellulose and hydroxypropylcellulose; binders such as gelatin, alginates, and polyvinyl pyrrolidone; lubricants such as talc, calcium stearate, hydrogenated castor oil, and polyethylene glycol; disintegrants such as povidone and crospovidone; and surfactants such as polysorbates, cetyl alcohol, and glycerol. The pharmaceutically acceptable carriers and diluents may be biologically and physiologically compatible with the subject. Diluents include, but are not limited to, saline, aqueous buffers, solvents, and/or dispersion media. In addition, for example, injections may additionally include preservatives, analgesics, solubilizers, or stabilizers, and topical preparations may additionally include bases, excipients, lubricants, or preservatives.

The pharmaceutical composition of the present invention may be used in an unfrozen form or stored in a frozen state for subsequent use. If freezing is required, standard cryopreservatives (e.g., DMSO, glycerol, Epilife^{®} cell freezing medium (Cascade Biologics)) may be added to the cell population before freezing.

The composition of the present invention may contain 1.0×10³ to 1.0×10⁵ cells per ml, preferably 1.0×10⁵ cells.

The pharmaceutical composition of the present invention may be formulated and administered as a unit dosage form of pharmaceutical preparation suitable for administration into the body of a patient according to conventional methods in the pharmaceutical field, wherein the preparation may contain an effective amount for single or multiple administrations. Suitable dosage forms for this purpose include parenteral preparations such as injections, infusions, and transplants. In addition, the pharmaceutical composition may contain conventional pharmaceutically acceptable inert carriers and diluents. The pharmaceutically acceptable carriers and diluents may be biologically and physiologically compatible with the mesenchymal stromal cells and the recipients to be transplanted. The diluents may be, but are not limited to, saline, aqueous buffers, solvents, and/or dispersion media. Furthermore, for example, injectable formulations may further include preservatives, analgesics, solubilizers, or stabilizers, and topical formulations may further include bases, excipients, lubricants, or preservatives.

The composition of the present invention may be used in an unfrozen form or stored in a frozen state for subsequent use. If freezing is required, standard cryopreservatives (e.g., DMSO, glycerol, Epilife^{®} cell freezing medium (Cascade Biologics)) may be added to the cell population before freezing.

Furthermore, the composition may be transplanted and administered using administration methods commonly used in the art, preferably, but not limited to, direct engraftment or transplantation into the diseased site of a patient requiring treatment. Further, the administration may be non-surgical administration using a catheter or surgical administration, such as injection or transplantation after incision of the diseased site. The dosage may range from 5 x 10⁵ to 10⁸ per 60 kg adult or from 5 x 10⁵ to 10⁸ per administration. However, it should be understood that the actual dosage of the active ingredient should be determined in light of various relevant factors such as the disease to be treated, the severity of the disease, the route of administration, the patient's weight, age, and sex, and therefore, the dosage does not limit the scope of the present invention in any way.

The present invention also provides a pharmaceutical composition comprising epithelial progenitor cells or stem cells derived from the minor salivary gland ducts for use in the prevention or treatment of a salivary gland disease.

Further, the present invention also provides the use of epithelial progenitor cells or stem cells derived from minor salivary gland ducts in for the manufacture of a medicament for the prevention or treatment of salivary gland diseases.

The present invention provides a method for treating a salivary gland disease, comprising administering epithelial progenitor cells or stem cells derived from mature salivary gland ducts to a subject in need thereof.

The subject means any animal, including humans, monkeys, cows, horses, sheep, pigs, chickens, turkeys, quails, cats, dogs, mice, rats, rabbits or guinea pigs, in which a salivary gland disease has developed or may develop.

All technical terms used herein, unless otherwise defined, have the same meaning as commonly understood by those skilled in the art. While preferred methods and samples are described herein, similar or equivalent methods are also included within the scope of the present invention. The contents of all publications cited herein as references are incorporated herein by reference.

### [Advantageous Effects]

The present invention provides epithelial progenitor cells or stem cells derived from minor salivary gland ducts for therapeutic use, thereby safely alleviating inflammation within the salivary glands and improving saliva production, resulting in effective treatment of salivary gland diseases. Furthermore, unlike existing mesenchymal stem cell treatments, the composition of the present invention exhibits excellent efficacy by immediately and directly regenerating damaged areas, and has the advantage of maintaining its properties even when a simplified passage method or freezing-thawing techniques are applied.

### [Description of Drawings]

FIG. 1 shows the overall flow of obtaining, large-scale culturing, and confirming therapeutic efficacy of epithelial progenitor cells or stem cells derived from a minor salivary gland duct according to the present invention.
FIG. 2 shows epithelial progenitor cells or stem cells derived from the minor salivary gland duct cultured according to an embodiment of the present invention.
FIG. 3 demonstrates the self-renewal ability of the epithelial progenitor cells or stem cells derived from the minor salivary gland duct cultured according to an embodiment of the present invention even after long-term culture.
FIG. 4 shows the representative expression difference results compared to mesenchymal stem cells through flow cytometry analysis of the epithelial progenitor cells or stem cells derived from the salivary gland duct cultured according to an embodiment of the present invention.
FIG. 5 shows the gene expression level confirmed by performing quantitative nucleic acid amplification on the epithelial progenitor cells or stem cells derived from the salivary gland duct cultured according to an embodiment of the present invention.
FIG. 6 shows the organoid-generating ability of the epithelial progenitor cells or stem cells derived from the minor salivary gland duct cultured according to an embodiment of the present invention.
FIG. 7 shows the experimental results of immunofluorescence staining confirming protein expression of the epithelial progenitor cells or stem cells derived from the minor salivary gland duct cultured according to an embodiment of the present invention.
FIG. 8 shows the experimental results confirming the salivary secretion function of the salivary glands of Sjogren's syndrome-induced mice, in which the epithelial progenitor cells or stem cells derived from the minor salivary gland duct cultured according to an embodiment of the present invention were directly administered into the duct.
FIG. 9 shows the results of an autoantibody ELISA experiment on Sjogren's syndrome-induced mice in which the epithelial progenitor cells or stem cells derived from the minor salivary gland duct cultured according to an embodiment of the present invention were administered into the duct, and the results of an experiment to restore the expression of AQP5 gene and KRT5 gene in the salivary glands.
FIG. 10 shows the experimental results on body weight and salivary secretion function of salivary glands of groups of nonirradiated and irradiated mice with salivary gland hypofunction, in which the epithelial progenitor cells or stem cells derived from the minor salivary gland duct cultured according to an embodiment of the present invention were directly administered into the duct.
FIG. 11 shows the results of a histological staining experiment of mice with salivary gland hypofunction, in which the epithelial progenitor cells or stem cells derived from the minor salivary gland duct cultured according to an embodiment of the present invention were administered into the duct, and the results of an experiment to restore the expression of the AQP5 gene in the salivary glands.

### [Best Mode]

Examples are presented to facilitate understanding of the present invention. The following Examples are provided only to more easily understand the present invention, and the contents of the present invention are not limited by these Examples.

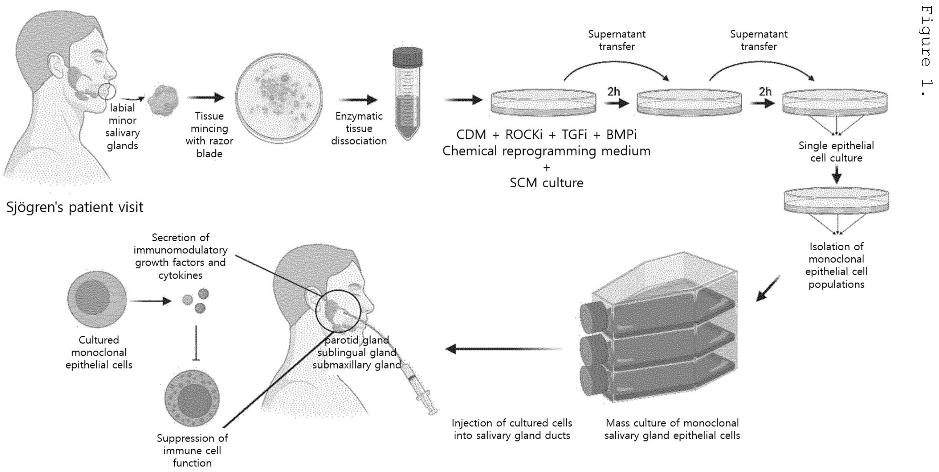

The experiments according to the present invention were conducted in the order indicated in FIG. 1. Specifically, epithelial progenitor cells or stem cells derived from the minor salivary gland ducts were obtained, and conditions for mass culture were established under xeno-free, serum-free, and feeder cell-free conditions. It was confirmed that salivary gland function was normalized by using the isolated monoclonal epithelial progenitor cells or stem cells derived from the minor salivary gland ducts.

### Example 1. Preparation of Monoclonal Epithelial Progenitor Cells or Stem Cells derived from Minor Salivary Gland Ducts

### 1. Reagents

The following reagents were used in the present invention.

DermaCult^{™}Keratinocyte Expansion Medium (# 100-0500, STEMCELL), DermaCult^{™}Keratinocyte Expansion Supplement (# 100-0502, STEMCELL), CnT-Prime Epithelial Proliferation Medium (# CnT-PR, CellnTec), Primocin (# anti-pm, Invivogen), Y-27632 dihydrochloride (# TB1254-GMP, Tocris), A83-01 (# TB2939-RMU, Tocris), DMH-1 (# 4126, Tocris), LDN 193189 dihydrochloride (# TB6053-GMP, Tocris), CTS^{™}DPBS, calcium, magnesium (# A1285801, Gibco), CTS^{™}DPBS (1×), without calcium chloride, without magnesium chloride (# A1285601, Gibco), Collagenase NB6 GMP grade (N0002779, Nordmark), CTS^{™}TrypLE^{™} Select Enzyme (# A1285901, Gibco), 100x20mm culture dish (# 150466, Thermo), 48 well culture plate (#30048, SPL), 24 well culture plate (#30024, SPL), 12 well culture plate (# 30012, SPL), 6 well culture plate (# 30006, SPL), 25cm² cell culture plate (# 70025, SPL), 75cm² cell culture plate (# 70075, SPL), 175cm² cell culture plate (# 70175, SPL), STEM-CELLBANKER-GMP Grade (# 11924, AMSBIO), Strainer (# 93070, SPL), Falcon^{®}5 mL Round Bottom Polystyrene Test Tube, with Cell Strainer Snap Cap (# 352235, Falcon), 1.5 mL Eppendorf tube, 5 mL conical Tube (# 51105, SPL), 15 mL conical Tube (# 51115, SPL), 50 mL conical Tube (# 51150, SPL).

### 2. Human Salivary Gland Epithelial Cells Obtained From Tissue

During surgical procedures such as tumor removal, normal tissue from the minor salivary glands that were not invaded by the tumor was collected through a biopsy or percutaneous needle biopsy, or 1-2 lobes were collected from the minor salivary glands of patients with suspected Sjogren's syndrome, placed in CTS^{™}DPBS, calcium, and magnesium, and stored in a 4°C refrigerator until the experiment was conducted on the same day or the next day. Collagenase was added to CTS^{™}DPBS, calcium, and magnesium to prepare a 0.4 U/mL solution, and Y27632 was added to the enzyme solution to a concentration of 10 µM. A volume of 1 mL was prepared for each 10 mg of tissue, and when the tissue weight was less than 10 mg, at least 1 mL was prepared. The tissue was placed on a petri dish, finely ground using a scalpel blade, added to the prepared enzyme solution, and reacted at 37°C for 1 hour. The reaction product was inverted once every 10 minutes to ensure that the solution and tissue were well mixed. The mixture was centrifuged at 300 g, 5 min, 4°C, and the enzyme solution was removed. Then, CTS^{™}DPBS (1×) without calcium chloride and magnesium chloride, was added in an amount twice the volume of the enzyme solution, and the mixture was centrifuged again at 300 g, 5 min, 4°C, and the DPBS was removed. CTS^{™}TrypLE^{™} Select Enzyme was added at 1 mL per 50 mg of raw tissue weight. Y27632 was added to a concentration of 10 µM (1 mL was added for tissues weighing less than 50 mg) and the resulting mixture was reacted at 37°C for 20 minutes, with inversion every 10 minutes to ensure even mixing. After mixing with an equal volume of culture medium, the mixture was passed through a 70 µm strainer and then through a Falcon tube with a strainer. The mixture was centrifuged at 300 g for 5 minutes at 4°C, and the supernatant was removed. The culture medium was then added again, and centrifuged at 300 g for 5 minutes at 4°C, and the supernatant was removed. Cells were counted.

### 3. Isolation of Human Salivary Gland Monoclonal Epithelial Cells

The cells were homogenized in 10 mL of culture medium, evenly distributed onto 100 x 20 mm culture dishes, and stored in an incubator at 37°C and 5% CO₂. Here, the composition of the culture medium was set as shown in [Table 1].

**[Table 1]**

| **Culture Medium 1** | **Final Concentration** | **Culture Medium 2** | **Final Concentration** |
|---|---|---|---|
| **Dermacult** | Basal Medium | **CnT-PR** | Basal Medium |
| **Dermacult Supplement** | 1X | **-** | - |
| **Prinmocin** | 100 µg/mL | **Prinmocin** | 100 µg/mL |
| **Y-27632** | 10 µM | **Y-27632** | 10 µM |
| **A83-01** | 1 µM | **A83-01** | 1 µM |
| **LDN193189** | 0.1 µM | **LDN193189** | 0.1 µM |

To isolate monoclonal epithelial cells by the subfractionation culturing method (SCM), the culture medium containing the cells was transferred to a new 100x20 mm culture dish after 2 hours, and this process was repeated twice more. Then, after 24 hours, the dish was re-cultured, and the old culture medium was removed and replaced with fresh culture medium at intervals of 2-3 days. After confirming colony formation, when the size of the cell cluster forming one colony exceeded 1 cm², the culture medium was removed and marked with a hydrophobic pen, and the remaining culture medium was removed using 1 x PBS. After adding 50-100 µL of TrypLE and Y27632, the cells were kept in an incubator at 37°C and 5% CO₂ for 15 minutes. The cell clusters detached from the dish using TrypLE were placed into separate 1.5 ml tubes, and 1 ml of culture medium was added to each tube. After centrifugation at 300 g and 4°C for 5 minutes, the supernatant was removed, and 250 µL of fresh culture medium was homogenized and dispensed into each well of a 48-well plate for each cell population.

### 4. Proliferation of Salivary Gland Epithelial Cells Isolated into Single-Cell Populations

The culture medium was removed every 2-3 days and replaced with fresh medium for subsequent culture. Subculture was performed when the cells in each well of the 48-well plate reached approximately 80-90% confluence. Here, the composition of the culture medium used for subculture was as shown in [Table 2]. After removing the culture medium, the remaining culture medium was removed with PBS. Then, TrypLE express (approximately 125 µl) was added to cover the bottom of the wells, and the cells were stored in an incubator at 37°C and 5% CO₂ for 15 minutes. Cells detached from the culture plate using TrypLE express were placed in separate 5 ml tubes, and the culture medium in [Table 2] was added to adjust the total volume to 1 mL and neutralized. After centrifugation at 300 g and 4°C for 5 minutes, the supernatant was removed, and 0.5 ml of the culture solution in [Table 2] was homogenized and dispensed into each well of a 24-well plate for each cell population. Then, the existing culture medium was removed every 2-3 days and replaced with fresh culture medium. The above subculture was scaled up as follows: 24-well culture plate (0.5 mL), 12-well culture plate (1 mL), 6-well culture plate (2 mL), 25 cm² cell culture flask (5 mL), 75 cm² cell culture flask (15 mL), and 175 cm² cell culture flask (35 mL). (The volume of culture medium added is indicated in parentheses.)

Cells from 6 wells were usable for various experiments, and when passaged, experiments were conducted at a ratio of 1:2 to 1:10. When population doubling was confirmed and the proliferation rate was not exponential, the cells were judged to be aged and discarded

**[Table 2]**

| **Culture Medium 1** | **Final Concentration** | **Culture Medium 2** | **Final Concentration** |
|---|---|---|---|
| **Dermacult** | Basal Medium | **CnT-PR** | Basal Medium |
| **Dermacult Supplement** | 1X | **-** | - |
| **Y-27632** | 10 µM | **Y-27632** | 10 µM |
| **A83-01** | 1 µM | **A83-01** | 1 µM |
| **LDN193189** | 0.1 µM | **LDN193189** | 0.1 µM |

### Example 2. Confirmation of Monoclonal Epithelial Progenitor Cells or Stem Cells derived from Minor Salivary Gland Ducts

The following experiments were performed when the clones successfully proliferated in Example 1 reached 70-80% confluency in culture medium.

Specifically, the morphological characteristics of the cells were examined using an optical microscope. Furthermore, the trypan blue exclusion method was used to confirm cell population doubling.

Furthermore, the expression of stem cell-related proteins on the cell surface was confirmed using flow cytometry.

In addition, the gene expression levels of the cells according to the present invention were confirmed using quantitative nucleic acid amplification.

Finally, differentiation potential was tested using three-dimensional culture using Matrigel.

### 1. Analysis of Cell Morphological Characteristics

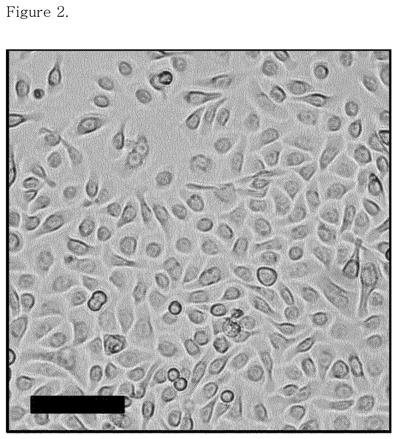

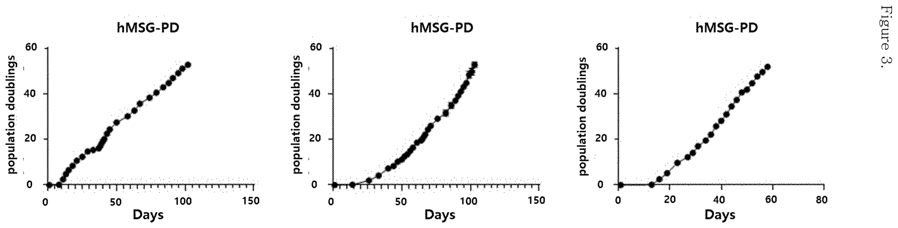

Before passage, morphological changes were observed using an optical microscope, and these changes are shown in FIG. 2. As shown in FIG. 2, it was confirmed that the cells of the present invention maintained a polygonal shape, a characteristic of epithelial progenitor cells or stem cells derived from the minor salivary gland ducts. Population doubling of the cells of the present invention was confirmed using the trypan blue exclusion method, demonstrating that the cells were capable of self-renewal even after long-term culture, as shown in FIG. 3.

### 2. Flow Cytometry Results

Flow cytometry was performed using cells that reached approximately 70-80% confluence. The culture medium was removed and the cells were washed with DPBS. TrypLE express was added, and the cells were maintained in an incubator at 37°C and 5% CO₂ for 15 minutes, and single-cell dissociation was confirmed. The cells detached using TrypLE express were neutralized by adding culture medium, centrifuged at 300g and 4°C for 5 minutes, the supernatant was removed, and the pellet was resuspended in DPBS. After filtering the cells into a FACS tube, the number of cells was measured and calculated to contain 1X10⁶ cells per tube. After centrifugation at 500 g and 4°C for 5 minutes, the cells were processed in a tube containing DPBS mixed with Zombie Violet Fixable Viability Dye, protected from light, and incubated at room temperature for 15 minutes. FACS buffer containing 0.5% BSA and 0.05% sodium azide in DPBS was added, and the cells were centrifuged at 500 g and 4°C for 5 minutes. Then, the supernatant was removed, and the resulting product were processed in a tube containing FACS buffer mixed with TruStain FcX, protected from light, and incubated at room temperature for 10 minutes. The cells were resuspended in a tube containing FACS buffer mixed with the fluorophore-conjugated antibodies, protected from light, and incubated at 4°C for 30 minutes. The cells were resuspended in FACS buffer, vortexed, and centrifuged twice at 500 g for 5 minutes at 4°C, followed by analysis on a flow cytometer.

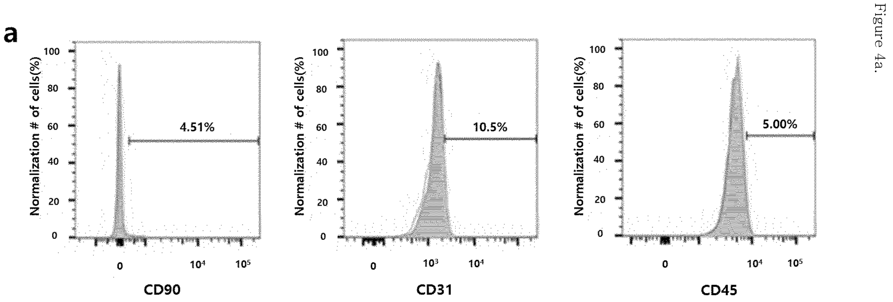

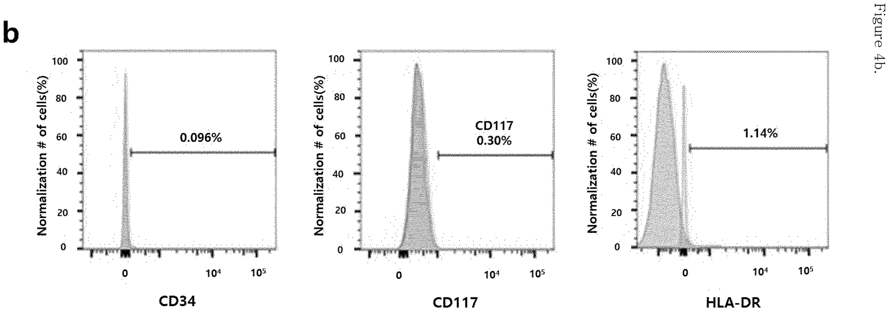

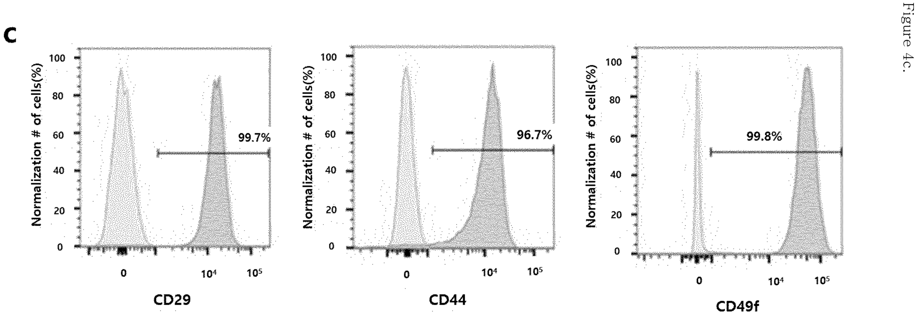

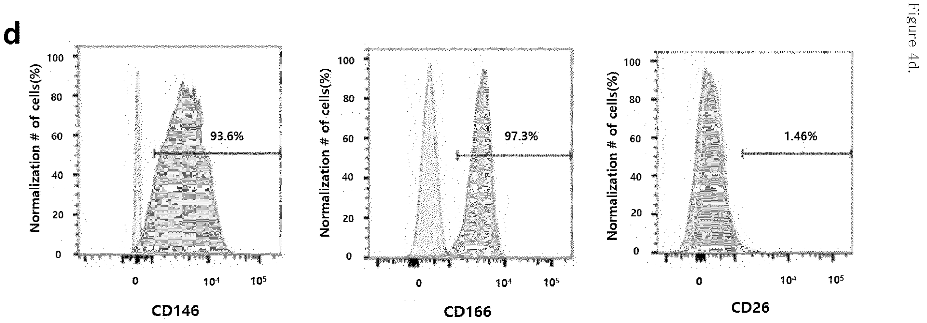

The results are shown in FIG. 4. As could be shown through FIG. 4, the cells according to the present invention exhibited the characteristics of high expression of CD29, CD44, CD49f, CD146, and CD166. These characteristics showed that the cells according to the present invention had the characteristics of salivary gland basal cells while still having high differentiation potential. On the other hand, luminal cell markers such as CD24, CD26, and CD117 were not expressed, and CD34 and CD90 were hardly expressed or had very low expression levels. In particular, the negative expression of CD34 and CD90 was different from the characteristics exhibited by mesenchymal stem cells (MSCs), confirming that the cells of the present invention exhibited a clear characteristic difference from MSCs.

### 3. Confirmation of Gene Expression using Quantitative Nucleic Acid Amplification

### (1) Reagents

TRIzol^{™} Reagent(Invitrogen^{™} #15596026), PrimeScript^{™} RT reagent Kit (Takara #RR037A), and SensiFAST^{™} SYBR^{®} Lo-ROX Kit (Bioline #BIO-94020)

### (2) Experimental Method

The culture medium was removed, then the cells were washed with 1 mL of DPBS in the culture dish and lysed with 1 mL of TRIZOL. RNA was isolated according to the manufacturer's protocol and dissolved in RNase-free deionized water. The RNA was quantified using a Nanodrop, and calculated to ensure that each tube contained 500 ng of RNA. Then, cDNA was synthesized using the PrimeScript^{™} RT Reagent Kit. Real-time PCR assays were then performed using the SensiFAST^{™} SYBR^{®} Lo-ROX Kit. The synthesis and analysis were performed according to the manufacturer's protocol.

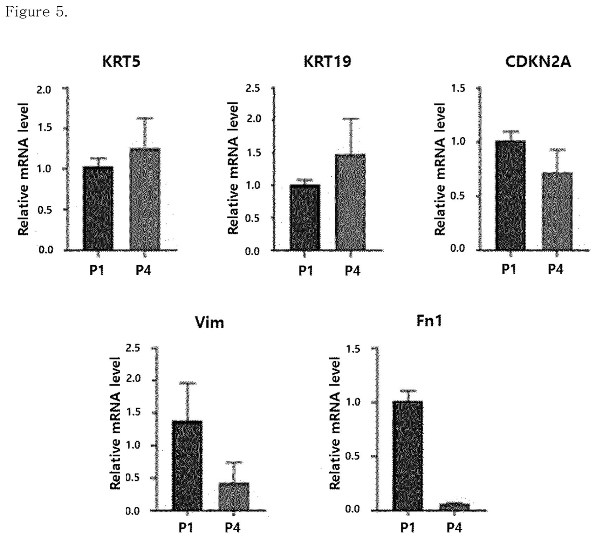

The results are shown in FIG. 5. As demonstrated in FIG. 5, the cells according to the present invention showed that the RNA of KRT5 and KRT19, which are salivary gland progenitor cell markers, increased after subculture. This characteristic showed that the cells according to the present invention have the characteristics of salivary gland epithelial progenitor cells or stem cells. In addition, the expression levels of CDKN2A RNA, which is an aging-related marker, decreased after subculture. This showed that the cells according to the present invention are able to be cultured for a long period of time without aging. In addition, the expression levels of Vimentin and Fibronectin-1 RNA, which are mesenchymal cell markers, decreased after subculture. These results showed that the cells according to the present invention have the characteristics of epithelial progenitor cells or stem cells.

### 4. Confirmation of Differentiation Potential through 3D Culture

### (1) Reagents for Confirmation of Differentiation Potential through 3D Culture

Advanced DMEM/F12 (ADF12) (#12634010, Gibco), HEPES (#15630-080, Gibco), GlutaMAX (#35050-061, Gibco), Y-27632 dihydrochloride (#1254, Tocris), 1×HBSS solution (#14025092, Gibco), Collagenase, type II (#4176, Worthington), Matrigel (#356231, Corning), TrypLE express (#12605-010, Life technologies), CellBanker 1 (Zenoaq), BSA (Bovine Serum Albumin; #BSA-68700-1kg, LSP), Primocin (#ant-pm, Invivogen), B-27 Supplement (50X), serum free (#17504-044, Gibco), NAC (N-acetylcysteine; #A9165, Sigma), Nicotinamide (#N0636, Sigma), A83-01 (#2939, Tocris), Prostaglandin E2 (#2296, Tocris), Recombinant RSPO3-Fc fusion protein conditioned medium (hRSPO3-CM) (R001, U-Protein Express BV), Noggin-Fc Fusion Protein conditioned medium (hNOG-CM) (#N002, U-Protein Express BV), Nrg1 (Neuregulin-1; #100-03, Peprotech), FGF2 (#100-18B, Peprotech), and FGF10 (#100-26, Peprotech).

### (2) Production and Culture Maintenance of Organoids Derived from Minor Salivary Glands of Patient with Sjogren's Syndrome

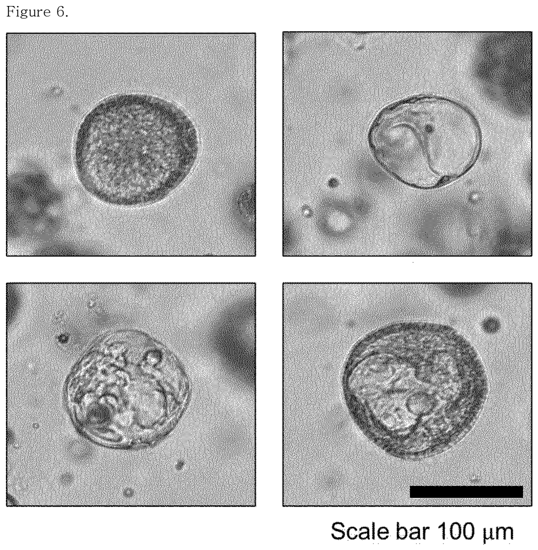

Minor salivary gland cells obtained from tissue stored in a nitrogen tank were thawed to slush, then 1,000 µL of Advanced DMEM/F12 was added and the cells were centrifuged at 500 g for 5 minutes. The supernatant was removed, and cells were seeded at a density of 3.0 x 10³ cells per well in a 24-well plate. After incubating the 24-well plate in a 37°C incubator for at least 30 minutes, the centrifuged cell pellet was mixed with 40 µL of Matrigel per well and placed in a dome shape into the well. The culture medium conditions for the organoids derived from the salivary gland of a patient with Sjogren's syndrome are shown in [Table 3]. To maintain the culture, the medium was replaced with fresh medium every 2-3 days. Before passage, the shape and size of the organoids were observed under an optical microscope, as shown in FIG. 6, confirming that organoid formation was maintained. The maintenance of formation and culture of the organoids enabled the confirmation of the differentiation potential of salivary gland-derived epithelial cells.

**[Table 3]**

| **Advanced DMEM/F12** | |
|---|---|
| **Glutamax** | 2 mM |
| **HEPES** | 10 mM |
| **Primocin** | 100 µg/mL |
| **B27 vit A+** | 1X |
| **NAC** | 1mM |
| **Nicotinamide** | 5mM |
| **A83-01** | 5µM |
| **PGE2** | 3µM |
| **hNOG-CM** | 2% |
| **hFGF2** | 5ng/mL |
| **hFGF10** | 10ng/mL |
| **hNRG1** | 5ng/mL |
| **hRSPO3-CM** | 1% |
| **Y-27632*** | 10µM |

| | |
|---|---|
| * Y-27632 was removed after 7 days of treatment from the start of organoid culture. | |

### Example 3. Confirmation of Protein Expression of Progenitor Cells derived from Minor Salivary Gland Ducts by Immunofluorescence Staining

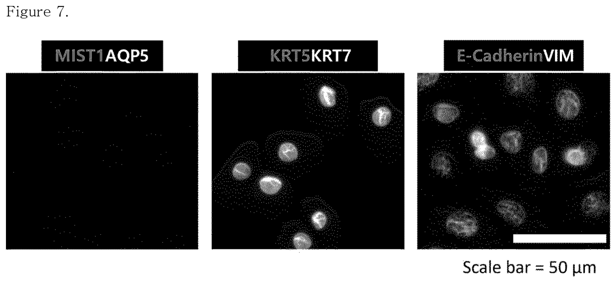

Epithelial progenitor or stem cell expression markers in the minor salivary gland ducts were identified by immunofluorescence staining using cells that had reached 70-80% proliferation. After removing the cell culture medium, the cells were washed with DPBS, and fixed for 10 minutes with 4% PFA or a 1:1 ratio of ethanol and methanol. After fixation, to prevent nonspecific binding, normal donkey serum was added and the cells were incubated for 1 hour. After washing with DPBS, the samples were incubated with the primary antibody overnight at 4°C, followed by incubation with the secondary antibody at room temperature for 1 hour. Then, mounting solution was then added, and the samples were covered with a coverslip and allowed to dry. Fluorescence microscopy revealed no expression of MIST1 or AQP5, markers of salivary gland acinar cells. KRT5, a salivary gland basal ductal cell marker, was expressed, but KRT, a ductal cell marker, was not expressed. Furthermore, it was confirmed that the epithelial cell marker E-Cadherin was expressed, while the mesenchymal stem cell marker Vimentin was not expressed, as shown in FIG. 7.

### Example 4. Confirmation of Therapeutic Efficacy in Mouse Model of Salivary Gland Disease

The therapeutic efficacy of Sjogren's syndrome-induced mice was confirmed by administering human minor salivary gland epithelial progenitor cells or stem cells according to the present invention into the salivary gland ducts.

Specifically, Sjogren's syndrome-induced mice (ESS) were created by mixing mouse salivary gland-derived antigens with an adjuvant and injecting the antigen-adjuvant mixture into allogeneic mice to induce an antigen-antibody response. Then, the cells according to the present invention were injected into the salivary gland ducts of mice via Wharton's duct using a catheter and a tube, and the therapeutic efficacy of the cells was confirmed.

Therapeutic efficacy was confirmed through saliva flow rate measurements and ELISA analysis of mouse blood.

To measure saliva flow rate, the weight of cotton to be placed in the mouse's mouth was measured in the experimental mouse model. Mice were then anesthetized by intraperitoneal injection of Ketamine (100 mg/kg) and Xylazine (15 mg/kg). Pilocarpine (0.5 mg/kg) was then injected intraperitoneally. Immediately following injection, cotton was placed in the mouse's mouth, and after five minutes, the cotton was removed and its weight was measured. Cotton was repeatedly placed in the mouse's mouth for a total of 25 minutes, and the secreted saliva was calculated by measuring and summing the weights.

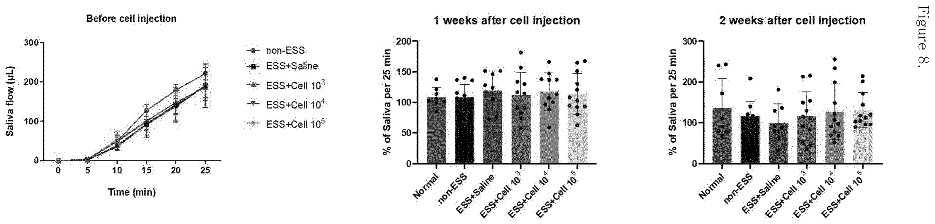

The results are shown in FIG. 8. As shown in FIG. 8, upon examination for a decrease in salivary secretion, which is a typical symptom of Sjogren's syndrome, it was confirmed that the treatment group injected with the cells according to the present invention showed a dose-dependent recovery in saliva secretion ability to the level of normal mice compared to when only saline solution, which is an excipient, was injected. These results demonstrated that the cells of the present invention exhibited excellent efficacy in restoring salivary gland function.

Next, changes in secreted factors were identified in mouse blood using ELISA analysis.

The experiment was conducted using the Mouse ANA ELISA Kit, 96T (#EM1607, Finetest) according to the manufacturer's protocol. At 14 days after cell administration, the experimental end point, the mice were euthanized with CO₂ gas. After an abdominal incision, blood was collected from the abdominal aorta using a 1 ml syringe. The supernatant was separated after centrifugation at 1,000 x g for 15 minutes, and the isolated sample was subjected to ELISA analysis according to the kit protocol.

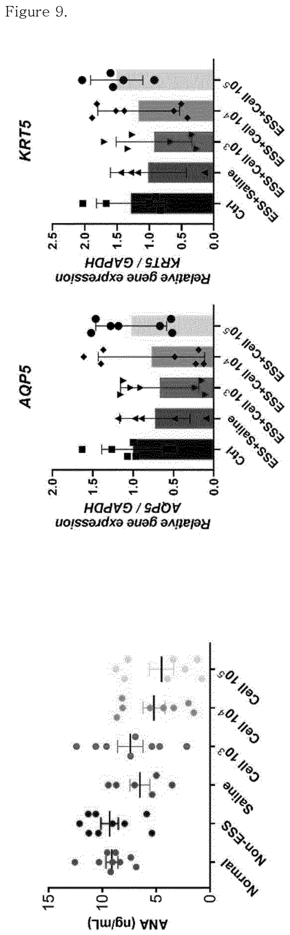

The results are shown in FIG. 9. It was confirmed from FIG. 9 that the ELISA analysis of autoantibodies in mouse blood revealed a dose-dependent decrease in autoantibody concentrations in the cell-injected group, confirming the effectiveness of the treatment for Sjogren's syndrome, an autoimmune disease. Furthermore, the cell-injected group showed a dose-dependent increase in the expression of the acinar marker AQP5 and the basal marker KRT5 in the salivary gland tissue compared to the control group, confirming the effect of salivary gland function recovery.

### Example 5. Confirmation of Therapeutic Efficacy in Mouse Model of Salivary Gland Hypofunction

A mouse model of salivary gland hypofunction induced by radiation was created, and the therapeutic efficacy was confirmed by administering cells according to the present invention into the salivary gland duct of the model.

Specifically, mice with salivary gland hypofunction were created by locally irradiating the salivary gland region of the mouse with a single 15 Gy dose of X-rays using a Biological X-Ray Irradiator (X-RAD) (Precision X-Ray Inc. (U.S.A)). The cells according to the present invention were then injected into the salivary gland ducts of the mice via Wharton's duct using a catheter and a tube, and the therapeutic efficacy of the cells was confirmed.

The therapeutic efficacy was confirmed by measuring saliva flow rates, histological staining, and quantitative nucleic acid amplification.

To measure saliva flow rate, the weight of cotton to be placed in the mouse's mouth was measured in the experimental mouse model. Mice were then anesthetized by intraperitoneal injection of Ketamine (100 mg/kg) and Xylazine (15 mg/kg). Pilocarpine (0.5 mg/kg) was then injected intraperitoneally. Immediately following injection, cotton was placed in the mouse's mouth, and after 15 minutes, the cotton was removed, its weight was measured, and the secreted saliva volume was calculated.

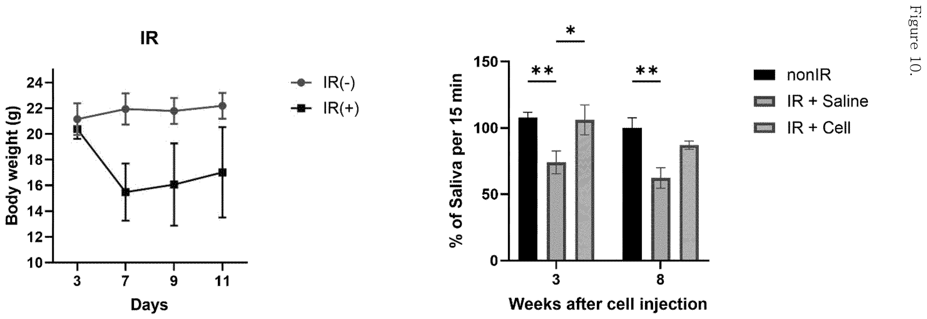

The results are shown in FIG. 10. Specifically, upon examination for a reduction in salivary secretion, which is one of the symptoms of salivary gland hypofunction, it was confirmed that the treatment group injected with the cells according to the present invention showed a dose-dependent recovery in saliva secretion ability to the level of normal mice compared to when only saline solution, which is an excipient, was injected. These results demonstrated that the cells of the present invention exhibited excellent efficacy in restoring salivary gland function.

Next, histological changes in the mouse salivary glands were examined using hematoxylin and eosin staining.

Experiments were performed using an H&E Staining Kit (#ab245880, Abcam) according to the manufacturer's protocol. At the end of the experiment, 21 days after cell administration, the mice were euthanized with CO₂ gas. The salivary gland was isolated by incision. The isolated salivary gland was fixed in 4% PFA for 24 hours, paraffin block was created, and paraffin sections were cut to a thickness of 5 µm. The salivary gland paraffin sections were subjected to histological staining according to the kit protocol.

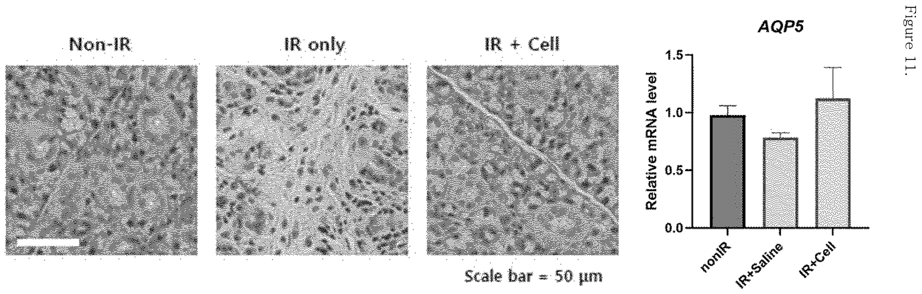

The results are shown in FIG. 11. The degree of damage to the salivary gland duct was assessed using staining in the mouse salivary glands, and as a result, it was shown that the cell-injected group showed a decrease in damage to the duct, confirming its efficacy in treating salivary gland hypofunction. Furthermore, the expression of AQP5, a marker of acinar cells, was increased in salivary gland tissue compared to the control group, confirming that the treatment group also had the salivary gland function recovery effect.

## Claims

1. A pharmaceutical composition for preventing or treating a salivary gland disease, comprising epithelial progenitor cells or stem cells derived from minor salivary gland ducts.

2. The pharmaceutical composition of claim 1, wherein the epithelial progenitor cells or stem cells derived from the minor salivary gland ducts are cells derived from minor salivary gland tissue.

3. The pharmaceutical composition of claim 1, wherein the epithelial progenitor cells or stem cells derived from the minor salivary gland ducts are cultured in the presence of a Rho-associated protein kinase (ROCK) inhibitor, a transforming growth factor-beta (TGF-β) inhibitor, and a bone morphogenetic protein (BMP) inhibitor.

4. The pharmaceutical composition of claim 3, wherein the ROCK inhibitor is Y-27632, Fasudil, or H-1152.

5. The pharmaceutical composition of claim 3, wherein the TGF-beta inhibitor is any one selected from the group consisting of A83-01, SB-431542, SB-505124, SB-525334, SD-208, RepSOX, LY-36494, and SJN-2511.

6. The pharmaceutical composition of claim 3, wherein the BMP inhibitor is any one selected from the group consisting of Noggin, Dorsomorphin, Gremlin 1, DMH1, and LDN-193189.

7. The pharmaceutical composition of claim 3, wherein the ROCK inhibitor is Y-27632, the TGF-beta inhibitor is A83-01, and the BMP inhibitor is DMH1 or LDN193189.

8. The pharmaceutical composition of claim 7, wherein the ROCK inhibitor is Y-27632, the TGF-beta inhibitor is A83-01, and the BMP inhibitor is LDN193189.

9. The pharmaceutical composition of claim 3, wherein the culturing is performed in an epithelial cell culture medium.

10. The pharmaceutical composition of claim 1, wherein the epithelial progenitor cells or stem cells derived from the minor salivary gland ducts highly express at least one cell surface marker selected from the group consisting of CD29, CD44, CD49f, CD146, CD166, KRT5, KRT19, and E-Cadherin, and do not express at least one cell surface marker selected from the group consisting of CD24, CD26, CD34, CD90, CD117, MIST, AQP5, KRT7, CDKN2A, Fibronectin-1, and Vimentin.

11. The pharmaceutical composition of claim 3, wherein the culturing is performed under xeno-free, serum-free, feeder-free, or all of these conditions.

12. The pharmaceutical composition of claim 1, wherein the epithelial progenitor cells or stem cells derived from the minor salivary gland ducts are obtained by:
(1) culturing cells derived from the minor salivary gland in a medium composition containing a ROCK inhibitor, a TGF-beta inhibitor, and a BMP inhibitor; and
(2) culturing and proliferating the epithelial progenitor cells or stem cells derived from the minor salivary gland ducts isolated from the culturing in Step (1) in a medium containing the ROCK inhibitor, the TGF-beta inhibitor, and the BMP inhibitor.

13. The pharmaceutical composition of claim 12, wherein the ROCK inhibitor is Y-27632, the TGF-beta inhibitor is A83-01, and the BMP inhibitor is DMH1 or LDN193189.

14. The pharmaceutical composition of claim 1, wherein the salivary gland disease is any one selected from the group consisting of xerostomia, infectious acute sialadenitis, infectious chronic sialadenitis, salivary gland tuberculosis, Sjogren's syndrome, sialolithiasis, salivary duct stenosis, sialadenosis, salivary gland tumors, salivary hypofunction due to aging, salivary hypofunction due to medication, and salivary hypofunction due to radiation therapy.

15. The pharmaceutical composition of claim 14, wherein the salivary gland disease is Sjogren's syndrome.

16. The pharmaceutical composition of claim 1, wherein the composition is administered into a salivary gland duct.

17. A method for preventing or treating a salivary gland disease, comprising administering a therapeutically effective amount of epithelial progenitor cells or stem cells derived from minor salivary gland ducts to a subject in need thereof.

18. The method of claim 17, wherein the salivary gland disease is any one selected from the group consisting of xerostomia, infectious acute sialadenitis, infectious chronic sialadenitis, salivary gland tuberculosis, Sjogren's syndrome, sialolithiasis, salivary duct stenosis, sialadenosis, salivary gland tumors, salivary hypofunction due to aging, salivary hypofunction due to medication, and salivary hypofunction due to radiation therapy.

19. The method of claim 18, wherein the salivary gland disease is Sjogren's syndrome.

20. Use of epithelial progenitor cells or stem cells derived from minor salivary gland ducts for the manufacture of a medicament for the prevention or treatment of salivary gland diseases.

21. The use of claim 20, wherein the salivary gland disease is any one selected from the group consisting of xerostomia, infectious acute sialadenitis, infectious chronic sialadenitis, salivary gland tuberculosis, Sjogren's syndrome, sialolithiasis, salivary duct stenosis, sialadenosis, salivary gland tumors, salivary hypofunction due to aging, salivary hypofunction due to medication, and salivary hypofunction due to radiation therapy.

22. The use of claim 21, wherein the salivary gland disease is Sjogren's syndrome.

23. A pharmaceutical composition comprising epithelial progenitor cells or stem cells derived from minor salivary gland ducts for use in the prevention or treatment of a salivary gland disease.

24. The pharmaceutical composition of claim 23, wherein the salivary gland disease is any one selected from the group consisting of xerostomia, infectious acute sialadenitis, infectious chronic sialadenitis, salivary gland tuberculosis, Sjogren's syndrome, sialolithiasis, salivary duct stenosis, sialadenosis, salivary gland tumors, salivary hypofunction due to aging, salivary hypofunction due to medication, and salivary hypofunction due to radiation therapy.

25. The pharmaceutical composition of claim 24, wherein the salivary gland disease is Sjogren's syndrome.
